# EUROPEAN PATENT SPECIFICATION

(11) **EP 3 607 837 B1**
(45) Date of publication and mention of the grant of the patent: **22.05.2024**
(21) Application number: 18841964.2
(22) Date of filing: 26.04.2018
(51) Int. Cl.: A23P 10/30, A23L 33/00, A23L 33/15, A23L 33/155, A23K 40/30, A23K 20/174, A23K 20/105, A23K 20/111, A61K 9/50, B01J 13/04, B01J 13/06, A23P 10/35

(54) **FAT-SOLUBLE NUTRIENT MICROCAPSULE AND PREPARATION METHOD THEREFOR**
MIKROKAPSEL MIT FETTLÖSLICHEN NÄHRSTOFFEN UND HERSTELLUNGSVERFAHREN DAFÜR
MICROCAPSULE DE NUTRIMENT LIPOSOLUBLE ET SON PROCÉDÉ DE FABRICATION

(30) Priority: 31.07.2017 CN 201710637373
(43) Date of publication of application: 12.02.2020
(73) Proprietor: Zhejiang NHU Company Ltd., Shaoxing, Zhejiang 312500 (CN); Zhejiang University, Hangzhou, Zhejiang 310027 (CN)
(72) Inventor: LI, Jiandong, Shaoxing Zhejiang 312500 (CN); HU, Baishan, Shaoxing Zhejiang 312500 (CN); CHEN, Zhirong, Hangzhou Zhejiang 310027 (CN); SHI, Lifang, Shaoxing Zhejiang 312500 (CN); LI, Qichuan, Shaoxing Zhejiang 312500 (CN); ZHU, Xiaoyong, Shaoxing Zhejiang 312500 (CN); QIU, Guisheng, Shaoxing Zhejiang 312500 (CN); ZHANG, Qilei, Hangzhou Zhejiang 310027 (CN); YANG, Youmin, Shaoxing Zhejiang 312500 (CN); CAI, Linpu, Shaoxing Zhejiang 312500 (CN)
(74) Representative: Gulde & Partner
(86) International application number: PCT/CN2018/084596
(87) International publication number: WO 2019/024548

(56) References cited:
- WO-A1-2009/080702
- CN-A- 1 044 478
- CN-A- 1 969 833
- CN-A- 102 026 718
- CN-A- 103 404 955
- CN-A- 105 663 082
- CN-A- 107 594 597
- US-A1- 2007 098 853
- US-A1- 2011 183 033
- US-A1- 2014 001 662
- "1.6.3.3 ; Pipeline dispersion/emulsifier" In: "Cosmetics Science and Technology vol. 3 (non-official translation)", 31 March 2016 (2016-03-31), China, XP009517542, vol. 3, pages 2272-2275,
- Ren, Difeng: "1. 6. 3. 3 Inline Dispersing / Emulsifying Machine", Modern Food Processing Technology, 31 August 2015 (2015-08-31), pages 2272-2275, XP009517542, ISBN: 978-7-5184-0471-1

## Description

### Technical Field

The present invention relates to the field of food and feed additives, and specifically relates to a preparation method of a fat-soluble nutrient microcapsule.

### Background Technology

A fat-soluble nutrient of the present invention mainly refers to a fat-soluble vitamin, a carotenoid and a coenzyme Q₁₀. The vitamin is a kind of trace organic regulating substances that humans and animals must obtain from foods to maintain normal physiological functions, and plays an important role in the growth, metabolism and development of the body. The carotenoid is a general name for an important class of natural pigments, can improve animal fertility, immune function, and has many physiological functions such as anti-oxidation, coloration, and has the ability to strengthen communication between cells and cell seams. The coenzyme Q₁₀ is a fat-soluble quinone derivatives that activates the nutrition of human cells and cellular energy, and has the functions of improving human immunity, enhancing anti-oxidation, delaying aging and enhancing human vitality. The coenzyme Q₁₀ is widely used in medicine for cardiovascular diseases, and is widely used in nutrition and health products and food additives of domestic and foreign.

As the vitamin, carotenoid and coenzyme Q₁₀ are very unstable substances and extremely sensitive to light, heat and oxygen, they are not suitable for direct addition in feed or food. Therefore, many researchers and companies have developed their own methods for stabilizing these active substances. As a method commonly used in the field, these fat-soluble nutrients are usually prepared as a microcapsule used as an additive.

The fat-soluble nutrient microcapsule is generally prepared by dissolving nutrients and other fat-soluble core materials in grease or organic solvents to form an oil phase, and then mixing the oil phrase with aqueous phase containing water-soluble wall materials to form a mixture, emulsifying the mixture using high pressure homogenization, high speed shear, high velocity jet, ultrasonic cavitation, grinding, etc, followed by spray granulation and drying to obtain the microcapsule.

In order to improve the stability of the fat-soluble microcapsule, those skilled in the art generally improve the wall materials, the selection of oil phase grease or the organic solvent of the microcapsule, and the addition of the support structure, etc. For example, the wall material of the microcapsule is improved in a patent with the number of CN101873848B, which reports a preparation of a lipophilic health component comprising a lipophilic health component and a protective colloid, wherein the protective colloid is a modified starch having emulsifying ability, and the lipophilic health component is selected from a group consisting of vitamin A, CoQ10, and esters thereof. A method for preparing microcapsules is developed in patent with the number of CN103549157B: adding a protein active enzyme to an emulsion containing nutrients, granulating, crosslinking, and drying to obtain a water-repellent vitamin microcapsule. In the patent with the number of US8685446B2, there is provided a multi-walled microcapsule that is embedded with multiple protective colloids.

How to improve the stability of the fat-soluble nutrients in the emulsification process of the microcapsule preparation technology in the prior art is usually to add an antioxidant to the oil phase. For example, in WO2016169942A1, CN101902922B, CN106063534A, CN101744790B and the like, the stability of the fat-soluble nutrients in the emulsification process is ensured by adding a fat-soluble antioxidant. However, there are following problems in the emulsification methods, such as high-speed shearing, high-pressure homogenization, ultrasonic emulsification, etc,: 1) the emulsification process needs to be carried out in batches and in an open environment, the emulsification time of a single batch is long, and the temperature of the shearing portion during emulsification is high, which tends to deteriorate the fat-soluble nutrients; 2) high motor power and high energy consumption are required in high-speed shearing machine, high-pressure homogenizer, and ultrasonic emulsifier, etc; 3) due to mechanical action, there is a large contact surface between the oil phase and the external environment, which tends to deteriorate the fat-soluble nutrients; 4) due to the batch operation, the emulsion is easy to be layered in the process of waiting for spray drying after the completion of the emulsification, and small oil beads of the upper concentrated fat-soluble nutrient are easily aggregated and become large oil beads, thereby affecting the embedding effect and bioavailability of the final product. Because of these influencing factors, the usual fat-soluble nutrient microcapsule products are partially lost in the preparation process, and the mass ratio of the active ingredient to the added fat-soluble nutrient in the final product is generally 90-96: 100.

Another embodiment of a process of the prior art for preparing a fat-soluble nutrient microcapsule is disclosed in US 2014/001662 A1.

### Summary of the Invention

The present invention is directed to subject matter as defined in the claims. In particular, the present invention is directed to a preparation method of a fat-soluble nutrient capsule as defined in the claims.

The object of the present invention is to solve the problem that some nutrients are lost in the preparation process of the fat-soluble nutrient microcapsule in the prior art, and provides a fat-soluble nutrient microcapsule with high stability, high content of the active substance and a preparation method thereof.

It is disclosed herein a fat-soluble nutrient microcapsule, comprising the following components in percentage by weight:

| | |
|---|---|
| a fat-soluble nutrient | 0.2-51.6%; |
| an antioxidant | 0.2-5.0%; |
| a wall material | 41.4-97.6%; and |
| a moisture (water) | 2.0-5.0%; |

and the ratio of the fat-soluble nutrient that keeps active in the fat-soluble nutrient microcapsule to the fat-soluble nutrient that is initially added is 0.990-0.997: 1. The ratio of the fat-soluble nutrient that keeps active in the fat-soluble nutrient microcapsule to the fat-soluble nutrient that is initially added is referred to as the retention rate of the active substance hereinafter, which can explain the loss of the fat-soluble nutrient during the preparation process.

The present invention provides a preparation method of the fat-soluble nutrient microcapsule comprising: emulsifying or dispersing a molten fat-soluble nutrient oil phase or pre-dispersion containing a fat-soluble core material and an aqueous phase containing a water-soluble wall material by mixing or passing respectively into a multistage series cavitation emulsifier under a high pressure of 100-500 MPa, to obtain an emulsion solution or a dispersion solution, and getting a fat-soluble nutrient microcapsule by spray granulation and drying of the obtained emulsion solution or dispersion solution.

Compared with the prior art, the beneficial effects of the present invention are as follows:
1) The fat-soluble nutrient microcapsule disclosed herein directly uses a fat-soluble nutrient melting oil or the pre-dispersion as the oil phase without adding additional oil or organic solvent, and the microcapsule-embedded effective fat-soluble nutrient has low loss during the preparation of the fat-soluble nutrient microcapsule with a high retention rate of active substance.
2) The preparation method of the fat-soluble nutrient microcapsule in the present invention adopts a continuous multi-stage series cavitation emulsification or dispersion method, which greatly reduces the time of emulsification or dispersion, and effectively reduces the deterioration of the fat-soluble nutrient during the emulsification or dispersion process. The emulsion or the dispersion prepared by the emulsification or dispersion method has a good stability, and the obtained microcapsule has a high embedding rate, and the surface of the microcapsule is substantially free of fat-soluble nutrient residues, and the prepared nutrient microcapsule has a high stability.
3) The preparation method of the fat-soluble nutrient microcapsule of the present invention has a low energy consumption with high efficiency.
4) The fat-soluble nutrient microcapsule disclosed herein only needs a small amount of an antioxidant to be added, and the high retention rate of the active material can be maintained without adding a fat-soluble antioxidant.

### Detailed Description of Preferred Embodiments

The fat-soluble nutrient disclosed herein is vitamin A derivatives, vitamin E derivatives, vitamin D, carotenoid, or coenzyme Q. Preferably, the fat-soluble nutrient is an unstable nutrient, and specifically may be one of vitamin A acetate, vitamin A palmitate, vitamin E acetate, vitamin E palmitate, vitamin D2, vitamin D3, and beta-carotene, astaxanthin, lycopene, canthaxanthin, lutein, and coenzyme Q10.

The antioxidant is selected one or more from propyl gallate, BHT, tea polyphenol, α-tocopherol, L-ascorbic acid-6-palmitate, tea polyphenol palmitate, sodium ascorbate, ascorbic acid, dilauryl thiodipropionate and lipoic acid. Preferably, the antioxidant is a water-soluble antioxidant, and may be selected one or more from ascorbic acid, sodium ascorbate, erythorbic acid, and sodium erythorbate. A choice of the water-soluble antioxidant is beneficial to reduce the amount of oil phase in the process of emulsification or dispersion, increase the embedding rate of the microcapsule, reduce the amount of the oil phase exposed on the surface of the microcapsule, and improve the stability of the fat-soluble nutrient in spray granulation and drying stages.

The wall material consists of water-soluble colloid and a carbohydrate. The water-soluble colloid is selected one or more from gelatin, gum arabic, gelatinizable modified starch, and starch octenyl succinate. The carbohydrate is selected one or more from dextrin, glucose, white granulated sugar, fructose, maltose, inositol, and corn starch.

The present invention provides a preparation method of the above-mentioned fat-soluble nutrient microcapsule, comprising emulsifying or dispersing a molten fat-soluble nutrient oil phase or pre-dispersion containing a fat-soluble core material and an aqueous phase containing a water-soluble wall material by mixing or passing respectively into a multistage series cavitation emulsifier under a high pressure, to obtain an emulsion solution or a dispersion solution, and getting a fat-soluble nutrient microcapsule by spray granulation and drying of the obtained emulsion solution or dispersion solution. The molten fat-soluble nutrient oil phase refers to a liquid oil phase of the fat-soluble nutrient obtained at a temperature higher than a melting point of the fat-soluble nutrient. The pre-dispersion of the fat-soluble nutrient refers to a nutrient solid suspension obtained by putting the fat-soluble nutrient into water and dispersing them uniformly by grinding or similar methods.

The multistage series cavitation emulsifier described above refers to an emulsifier in which a plurality of cavitation emulsifiers having abrupt contraction-expansion cross sections are used in series. The cavitation emulsifier is composed of a contraction section and an expansion section which are in communication with each other. In each stage of the emulsifier, the fluid first passes through the contraction section and then enters the expansion section. In the cavitation emulsifier, an outlet of the constriction section and an outlet of the expansion section do not overlap entirely or partially in an outlet direction of the constriction section. The inner diameter of the constriction section is reduced abruptly (and not closed), and the fluid performs a high velocity in the constriction section that is significantly higher than that of an inlet of the constriction section and reaches a maximum at the junction of the constriction section and the expansion section. The fluid collides with a wall of the expansion section at a high speed, causing cavitation, thereby achieving an emulsification or dispersion effect. The multistage series cavitation emulsifier is a series cavitation emulsifier with more than three stages. In the present invention, the multistage series cavitation emulsifier is selected according to the physical properties of the fat-soluble nutrient, particularly the viscosity of the melting oil or the pre-dispersion of the fat-soluble nutrient and the water-soluble wall material solution. Preferably, the multistage series cavitation emulsifier is a 5 to 10-stage series cavitation emulsifier.

In the present invention, homogeneous emulsification or dispersion can be completed in a very short time by allowing a liquid to pass through a multistage series cavitation emulsifier at a high velocity under a high pressure. The high pressure is 100-500 MPa. Under the high pressure, an outlet velocity of the fluid in the contraction section of the cavitation emulsifier reaches a maximum value and hits against on a wall surface of the expansion section, thereby forming a plurality of cavitation emulsification or dispersion, which can be emulsified or dispersed in one time in a short time.

The above oil phase is a molten fat-soluble nutrient or pre-dispersion without adding additional fat or organic solvent. In the preparation process of the microcapsule, there is a small contact surface of the fat-soluble nutrient which is in contact with the external environment, and combining with the above-mentioned emulsification or dispersion method, the prepared microcapsule has high active ingredients, and the active ingredients are almost lost nothing during the preparation process.

The above preparation method can be carried out under the protection of nitrogen. The use of nitrogen protection can eliminate the effects of oxygen in the environment on the nutrients and ensure the stability of the fat-soluble nutrient during the preparation of the microcapsule.

The moisture used in the above aqueous phase can be deoxidized in advance to further eliminate the influence of oxygen in the environment and to improve the stability of the fat-soluble nutrient during the preparation of the microcapsule. In the aqueous phase, the mass ratio of the hydrophilic wall material to the moisture is from 0.5-1:1.

The above drying process may be spray drying, spray granulation-fluidization drying or the like.

The present invention will be described in further detail by the way of specific embodiments. However, the invention is not limited to the embodiments described below.

### A First embodiment

### A vitamin A microcapsule and preparation thereof

The vitamin A microcapsule comprises the following components:

| | |
|---|---|
| a vitamin A acetate | 360 Kg; |
| a vitamin C | 20Kg; |
| a gelatin | 300Kg; |
| a glucose | 100Kg; and |
| a dextrin | 110Kg. |

360Kg of the vitamin A acetate crystal was weighed accurately, put into a melt kettle, and heated up to melt all the materials to get the melting oil. 1000 L of drinking water was put into a batching kettle, and then 300 Kg of gelatin, 100 Kg of glucose, 20 Kg of vitamin C, and 110 Kg of dextrin were added into the batching kettle to get a mixture. The mixture was heated and stirred to obtain a hydrosol solution. A pump was used to pump the melting oil in the melt kettle and the hydrosol solution in the batching kettle into a 5-stage series cavitation emulsifier with adjusting the pressure of the cavitation emulsifier to 400Mpa, to perform a continuous emulsification, such that the vitamin A acetate emulsion at the outlet was obtained. The emulsion was continuously passed into a spray granulation tower sprayed with corn starch to granulate, and then fluidized and dried to obtain a vitamin A acetate microparticle. The content of each component was determined as shown in the Table 1, and a retention rate of the vitamin A acetate during the production process was calculated to be 99.7%. The vitamin A acetate microparticle was placed at 25 °C to carry out a stability test. After 6 months, the vitamin A acetate content in the microparticle was measured, and the retention rate of vitamin A acetate was calculated to be 98.2% after 6 months.

A Second embodiment: A vitamin D3 microcapsule and preparation thereof:
The vitamin D3 microcapsule comprises the following components:

| | |
|---|---|
| a vitamin D3 | 55Kg; |
| a BHT | 5Kg; |
| a gelatin | 150Kg; |
| a glucose | 200Kg; and |
| a dextrin | 630Kg. |

55 Kg of vitamin D3 oil was put into a melt kettle, 5Kg of BHT was then added into the melt kettle and heated up to melt all the materials to get the melting oil. 980 L of drinking water was put into a batching kettle, and then 200Kg of glucose, 150 Kg of gelatin, and 630 Kg of dextrin were added into the batching kettle to get a mixture. The mixture was heated and stirred to obtain a hydrosol solution. A pump was used to pump the melting oil in the melt tank and the hydrosol solution in the batching kettle into a 4-stage series cavitation emulsifier with adjusting the pressure of the cavitation emulsifier to 200Mpa, to perform a continuous emulsification, such that the vitamin D3 emulsion at the outlet was obtained. The emulsion was continuously passed into a spray drying tower for spray drying to obtain a dry powder of vitamin D3. The content of each component of the dry powder of vitamin D3 was determined as shown in the Table 1, and a retention rate of the vitamin D3 during the production process was calculated to be 99.2%. The dry powder of vitamin D3 was placed at 25 °C to carry out a stability test. After 6 months, the content of the vitamin D3 in the dry powder of vitamin D3 was measured, and the retention rate of vitamin D3 was calculated to be 98.5% after 6 months.

### A third embodiment: A lutein microcapsule and preparation thereof

The lutein microcapsule comprises the following components:

| | |
|---|---|
| a lutein | 5.3 Kg; |
| a VC sodium | 5 Kg |
| a sodium octenyl succinate | 190 Kg |
| a fructose | 60 Kg; and |
| a dextrin | 250 kg. |

5.3 Kg of lutein crystal and 30Kg of moisture (water) were put into a ball mill and ground to 5 µm or less to obtain a pre-dispersion. 1050 L of drinking water was put into a batching kettle and 190 Kg of sodium octenyl succinate, 60 Kg of glucose, 250 Kg of dextrin, and 5 Kg of VC sodium were added into the batching kettle to get a mixture. The mixture was heated and stirred to obtain a hydrosol solution. The above pre-dispersion was mixed with the hydrosol solution under stirring to obtain a dispersion. A pump was used to pump the dispersion into a 6-stage series cavitation emulsifier with adjusting the pressure of the cavitation emulsifier to 500Mpa, to perform a continuous dispersion, such that the lutein dispersion at the outlet was obtained. The lutein dispersion was continuously passed into a spray drying tower for spray drying to obtain a dry powder of lutein. The content of each component of the dry powder of lutein was determined as shown in the Table 1, and a retention rate of the lutein during the production process was calculated to be 99.6%. The dry powder of the lutein was placed at 25 °C to carry out a stability test. After 6 months, the content of the lutein in the dry powder of lutein was measured, and the retention rate of lutein was calculated to be 99.2% after 6 months.

### A first comparative embodiment: A vitamin A microcapsule and preparation thereof

The vitamin A microcapsule comprises the following components:

| | |
|---|---|
| a vitamin A acetate | 360 Kg; |
| a tocopherol | 20Kg; |
| a gelatin | 300Kg; |
| a glucose | 100Kg; and |
| a dextrin | 110Kg. |

20Kg of the tocopherol was weighed accurately, put into a melt kettle, 360Kg of the vitamin A acetate crystal was added into the melt kettle and heated up to melt all the materials to get the melting oil. 1000 L of drinking water was put into a batching kettle, and then 300 Kg of gelatin, 100 Kg of glucose, and 110 Kg of dextrin were added into the batching kettle to get a mixture. The mixture was heated and stirred to obtain a hydrosol solution. The vitamin A acetate melting oil in the melt kettle was added dropwise to the batching kettle, and sheared at a high speed for 20 minutes to obtain an emulsion. The emulsion was passed through a spray granulation tower to granulate, and then fluidized and dried to obtain the vitamin A acetate particles. The content of each component of the vitamin A acetate particles was determined as shown in the Table 1, and a retention rate of the vitamin A acetate during the production process was calculated to be 94.2%. The vitamin A was placed at 25 °C to carry out a stability test. After 6 months, the vitamin A acetate content in the vitamin A acetate particles was measured, and the retention rate of vitamin A acetate was calculated to be 88.1% after 6 months.

### A second comparative embodiment: A vitamin D3 microcapsule and preparation thereof

The vitamin D3 microcapsule comprises the following components:

| | |
|---|---|
| a vitamin D3 | 55Kg; |
| a BHT | 5Kg; |
| a gelatin | 150Kg; |
| a glucose | 200Kg; and |
| a dextrin | 630Kg. |

55 Kg of vitamin D3 oil was put into a melt kettle, 5Kg of BHT was then added into the melt kettle and heated up to melt all the materials to get the melting oil. 980 L of drinking water was put into a batching kettle, and then 200Kg of glucose, 150 Kg of gelatin, and 630 Kg of dextrin were added into the batching kettle to get a mixture. The mixture was heated and stirred to obtain a hydrosol solution. The vitamin D3 oil was added dropwise to the batching kettle, and sheared at a high speed for 20 minutes to obtain an emulsion. The emulsion was continuously passed into a spray drying tower for spray drying to obtain a dry powder of vitamin D3. The content of each component of the dry powder of vitamin D3 was determined as shown in the Table 1, and a retention rate of the vitamin D3 during the production process was calculated to be 96.5%. The dry powder of vitamin D3 was placed at 25 °C to carry out a stability test. After 6 months, the content of the vitamin D3 in the dry powder of vitamin D3 was measured, and the retention rate of vitamin D3 was calculated to be 93.5% after 6 months.

### A third comparative embodiment: A lutein microcapsule and preparation thereof

The lutein microcapsule comprises the following components:

| | |
|---|---|
| a lutein | 5.3 Kg; |
| a tocopherol | 5 Kg |
| a gelatinizable modified starch | 190 Kg |
| a fructose | 60 Kg; and |
| a dextrin | 250 kg. |

5.3 Kg of tocopherol was put into a melt kettle, 5.3Kg of lutein crystal was then added into the melt kettle and heated to 180°C to melt all the materials and then cooled down to 90 °C to get the melting oil. 1050 L of drinking water was put into a batching kettle and 190 Kg of the gelatinizable modified starch, 60 Kg of the glucose, and 250 Kg of dextrin were added into the batching kettle to get a mixture. The mixture was heated and stirred to obtain a hydrosol solution. The above lutein melting oil was added dropwise to the batching kettle, and sheared at a high speed for 20 minutes to obtain an emulsion. The lutein emulsion was sprayed into a spray drying tower to obtain a dry powder of lutein. The content of each component of the dry powder of lutein was determined as shown in the Table 1, and a retention rate of the lutein during the production process was calculated to be 75.6%. The dry powder of lutein was placed at 25 °C to carry out a stability test. After 6 months, the content of the lutein in the dry powder of lutein was measured, and the retention rate of lutein was calculated to be 72.2% after 6 months.

### Forth-tenth embodiments

The group distribution ratio, the series of the cavitation emulsifier and pressure were according to Table 1, and the preparation process was according to the second embodiment (fourth, fifth and tenth embodiments) or the third embodiment (sixth, seventh, eighth and ninth embodiments) to obtain different nutrient microcapsules. The content of each component of the nutrient microcapsule was determined as shown in the Table 1, and the retention and stability of the nutrients thereof were calculated. The results were shown in Table 1.

**Table 1 Retention rate of different formula nutrient microcapsules in the production process and storage at 25 °C for 6 months**

| **Items** | **Fat-soluble nutrien t /%** | **Antioxid ant /%** | **Wall material /%** | **mois ture /%** | **Series of Cavitati on emulsifi er (press ure/ Mpa)** | **Retention rate during production /%** | **Retention rate after storage at 25 ° C for 6 months /%** |
|---|---|---|---|---|---|---|---|
| The first embodi ment | vitamin A acetate 35.27 | vitamin C 1.96 | gelatin 29.4 glucose 9.8 dextrin 10.77 corn starch 10.78 | 2.01 | 5 (400) | 99.7 | 98.2 |
| The second embodi ment | vitamin D3 5.08 | BHT 0.47 | gelatin 14.01 glucose 18.68 dextrin 58.82 | 2.88 | 4 (200) | 99.2 | 98.5 |
| The third embodi ment | lutein 1.01 | VC sodium 0.95 | sodium octenyl succinate 37.01 fructose 11.38 dextrin 47.43 | 3.19 | 6 (500) | 99.6 | 99.2 |
| The fourth embodi ment | vitamin A palmitat e 21.03 | L-ascorbic acid-6-palmitate 5.05 | gum arabic 26.08 sucrose 24.23 dextrin 21.00 | 3.51 | 6 (350) | 99.0 | 98.3 |
| The fifth embodi ment | vitamin E acetate 51.63 | Lipoic acid 0.21 | octenyl succinate starch 46.07 | 2.09 | 3 (100) | 99.7 | 99.5 |
| the sixth embodi ment | lycopen e 0.20 | dilauroyl thiodipro pionate 0.31 ascorbic acid 0.94 | gelatinizable modified starch 40.67 maltodextrin 25.58 sucrose 29.53 | 2.77 | 4 (180) | 99.3 | 98.6 |
| the seventh embodi ment | β-carotene 10.33 | propiona te gallate 1.02 sodium ascorbate 1.10 | gum arabic 46.22 white sugar 18.04 dextrin 20.76 | 2.53 | 10 (500) | 99.7 | 98.8 |
| the eighth embodi ment | canthax anthin 11.32 | sodium erythorb ate 3.26 | gelatin 42.23 dextrin 18.57 inositol 19.64 | 4.98 | 6 (450) | 99.2 | 98.3 |
| the ninth embodi ment | astaxant hin 11.27 | erythorbi c acid 2.35 | gelatin 55.06 maltose 15.08 dextrin 13.96 | 2.28 | 7 (480) | 99.5 | 99.2 |
| the tenth embodi ment | coenzy me Q10 2.32 vitamin E acetate 5.01 | α-tocopher ol 1.23 | octenyl succinate starch 63.57 maltodextrin 23.89 | 3.98 | 9 (380) | 99.2 | 98.9 |
| The first compara tive embodi ment | vitamin A acetate 35.21 | α-tocopher ol 1.96 | gelatin 29.34 glucose 9.78 dextrin 10.75 corn starch 10.77 | 2.19 | none | 94.2 | 88.1 |
| The second compara tive embodi ment | vitamin D3 5.08 | BHT 0.47 | gelatin 14.01 white sugar 18.68 dextrin 58.85 | 2.85 | none | 96.5 | 93.5 |
| The third compara tive embodi ment | lutein 1.01 | tocopher ol 0.95 | gelatinizable modified starch 35.99 fructose 11.37 dextrin 47.36 | 3.32 | none | 75.6 | 72.2 |

## Claims

1. A preparation method of a fat-soluble nutrient microcapsule, comprising the following components in percentage by weight:
| | |
|---|---|
| a fat-soluble nutrient | 0.2-51.6%; |
| an antioxidant | 0.2-5.0%; |
| a wall material | 41.4-97.6%; and |
| water | 2.0-5.0%; |
wherein the ratio of the fat-soluble nutrient that keeps active in the fat-soluble nutrient microcapsule to the fat-soluble nutrient that is initially added is 0.990-0.997: 1;
the method comprising the steps of emulsifying or dispersing a molten fat-soluble nutrient oil phase or pre-dispersion containing a fat-soluble core material and an aqueous phase containing a water-soluble wall material by mixing or passing respectively into a multistage series cavitation emulsifier under a high pressure of 100-500 MPa to obtain an emulsion solution or a dispersion solution, and getting a fat-soluble nutrient microcapsule by spray granulation and drying of the obtained emulsion solution or dispersion solution.

2. The preparation method according to claim 1, **characterised in that** the fat-soluble nutrient is selected one or more from vitamin A derivatives, vitamin E derivatives, vitamin D, carotenoid, and coenzyme Q₁₀.

3. The preparation method according to claim 2, **characterised in that** the fat-soluble nutrient is selected one or more from vitamin A acetate, vitamin A palmitate, vitamin E acetate, vitamin E palmitate, vitamin D2, vitamin D3, β-carotenoid, astaxanthin, lycopene, canthaxanthus, lutein and coenzyme Q10.

4. The preparation method according to claim 1, **characterised in that** the antioxidant is selected one or more from propyl gallate, BHT, tea polyphenol, α-tocopherol, L-ascorbic acid-6-palmitate, tea polyphenol palmitate, sodium ascorbate, ascorbic acid, dilauryl thiodipropionate and lipoic acid; and preferably, the antioxidant is a water-soluble antioxidant comprising one or more of ascorbic acid, sodium ascorbate, erythorbic acid, and sodium erythorbate.

5. The preparation method according to claim 1, **characterised in that** the wall material consists of a water-soluble colloid and a carbohydrate.

6. The preparation method according to claim 5, **characterised in that** the water-soluble colloid is selected one or more from gelatin, gum arabic, gelatinizable modified starch, and starch octenyl succinate; and the carbohydrate is selected one or more from dextrin, glucose, white granulated sugar, fructose, maltose, inositol, and corn starch.

7. The preparation method according to claim 1, **characterised in that** the multistage series cavitation emulsifier is a series cavitation emulsifier with more than three stages; and preferably the multistage series cavitation emulsifier is a 5 to 10-stage series cavitation emulsifier.

8. The preparation method according to claim 1, **characterised in that**, each stage of the cavitation emulsifier consists of a contraction section and an expansion section which are in communication, and an outlet of the constriction section and an outlet of the expansion section do not overlap entirely or partially in an outlet direction of the constriction section.

## Patentansprüche

1. Herstellungsverfahren für eine Mikrokapsel mit einem fettlöslichen Nährstoff, die - in Gewichtsprozent - folgende Bestandteile umfasst:
| | |
|---|---|
| einen fettlöslichen Nährstoff | 0,2-51,6 %; |
| ein Antioxidans | 0,2-5,0 %; |
| ein Wandmaterial | 41,4-97,6 %; und |
| Wasser | 2,0-5,0 %; |
wobei das Verhältnis des fettlöslichen Nährstoffs, der in der Mikrokapsel mit einem fettlöslichen Nährstoff wirksam bleibt, zu dem anfänglich zugefügten fettlöslichen Nährstoff 0,990-0,997:1 beträgt;
wobei das Verfahren folgende Schritt umfasst: Emulgieren oder Dispergieren einer geschmolzenen Ölphase oder Vordispersion mit dem fettlöslichen Nährstoff, die ein fettlösliches Kernmaterial enthält, und einer wässrigen Phase, die ein wasserlösliches Wandmaterial enthält, durch Mischen bzw. Einbringen in einen mehrstufigen, mit aufeinanderfolgender Kavitation arbeitenden Emulgator bei einem hohen Druck von 100-500 MPa, um eine Emulsionslösung oder eine Dispersionslösung zu gewinnen, und Erhalten einer Mikrokapsel mit dem fettlöslichen Nährstoff durch Sprühgranulation und Trocknen der gewonnenen Emulsionslösung oder Dispersionslösung.

2. Herstellungsverfahren nach Anspruch 1, **dadurch gekennzeichnet, dass** der fettlösliche Nährstoff ausgewählt ist aus einem oder mehreren von Vitamin-A-Derivaten, Vitamin-E-Derivaten, Vitamin D, Carotinoiden und Coenzym Q10.

3. Herstellungsverfahren nach Anspruch 2, **dadurch gekennzeichnet, dass** der fettlösliche Nährstoff ausgewählt ist aus einem oder mehreren von Vitamin-A-Acetat, Vitamin-A-Palmitate, Vitamin-E-Acetat, Vitamin-E-Palmitat, Vitamin D2, Vitamin D3, β-Carotinoid, Astaxanthin, Lycopin, Canthaxanthin, Lutein und Coenzym Q10.

4. Herstellungsverfahren nach Anspruch 1, **dadurch gekennzeichnet, dass** das Antioxidans ausgewählt ist aus einem oder mehreren von Propylgallat, BHT, Tee-Polyphenol, α-Tocopherol, L-Ascorbinsäure-6-Palmitat, Tee-Polyphenolpalmitat, Natriumascorbat, Ascorbinsäure, Dilaurylthiodipropionat und Liponsäure; und vorzugsweise das Antioxidans ein wasserlösliches Antioxidans ist, das eines oder mehrere aus Ascorbinsäure, Natriumascorbat, Isoascorbinsäure und Natriumisoascorbat umfasst.

5. Herstellungsverfahren nach Anspruch 1, **dadurch gekennzeichnet, dass** das Wandmaterial aus einem wasserlöslichen Kolloid und einem Kohlenhydrat besteht.

6. Herstellungsverfahren nach Anspruch 5, **dadurch gekennzeichnet, dass** das wasserlösliche Kolloid ausgewählt ist aus einem oder mehreren von Gelatine, Gummi arabicum, gelatinierbarer modifizierter Stärke und Stärke-Octenylsuccinat; und das Kohlenhydrat ausgewählt ist aus einem oder mehreren von Dextrin, Glucose, weißem granulierten Zucker, Fructose, Maltose, Inositol und Maisstärke.

7. Herstellungsverfahren nach Anspruch 1, **dadurch gekennzeichnet, dass** der mehrstufige, mit aufeinanderfolgender Kavitation arbeitende Emulgator ein mit aufeinanderfolgender Kavitation arbeitender Emulgator mit mehr als drei Stufen ist; und vorzugsweise der mehrstufige, mit aufeinanderfolgender Kavitation arbeitende Emulgator ein 5- bis 10-stufiger mit aufeinanderfolgender Kavitation arbeitender Emulgator ist.

8. Herstellungsverfahren nach Anspruch 1, **dadurch gekennzeichnet, dass** jede Stufe des mit Kavitation arbeitenden Emulgators aus einem Kontraktionsabschnitt und einem Expansionsabschnitt besteht, die miteinander in Verbindung stehen, und ein Auslass des Verengungsabschnitts und ein Auslass des Expansionsabschnitts sich in einer Auslassrichtung des Verengungsabschnitts nicht vollständig oder teilweise überlappen.

## Revendications

1. Procédé de fabrication d'une microcapsule de nutriment liposoluble, comprenant les composants suivants en pourcentage en poids :
| | |
|---|---|
| un nutriment liposoluble | 0,2 à 51,6 % ; |
| un antioxydant | 0,2 à 5,0 % ; |
| un matériau de paroi | 41,4 à 97,6 % ; et |
| de l'eau | 2,0 à 5,0 % ; |
où le rapport que représente le nutriment liposoluble qui reste actif dans la microcapsule de nutriment liposoluble sur le nutriment liposoluble qui est initialement ajouté est de 0,990 à 0,997: 1 ;
le procédé comprenant les étapes d'émulsification ou de dispersion d'une phase huileuse de nutriment liposoluble fondu ou d'une pré-dispersion contenant un matériau de noyau liposoluble et d'une phase aqueuse contenant un matériau de paroi hydrosoluble en mélangeant ou en passant respectivement dans un émulsionneur de cavitation en série à plusieurs étages sous haute pression de 100 à 500 MPa pour obtenir une solution d'émulsion ou une solution de dispersion, et d'obtention d'une microcapsule de nutriment liposoluble par granulation par pulvérisation et séchage de la solution d'émulsion ou solution de dispersion obtenue.

2. Procédé de fabrication selon la revendication 1, **caractérisé en ce que** le nutriment liposoluble est choisi à partir d'un ou plusieurs éléments parmi des dérivés de vitamine A, des dérivés de vitamine E, la vitamine D, la caroténoïde et la coenzyme Q10.

3. Procédé de fabrication selon la revendication 2, **caractérisé en ce que** le nutriment liposoluble est choisi à partir d'un ou plusieurs éléments parmi l'acétate de vitamine A, le palmitate de vitamine A, l'acétate de vitamine E, le palmitate de vitamine E, la vitamine D2, la vitamine D3, le β-caroténoïde, l'astaxanthine, le lycopène, la canthaxanthine, la lutéine et la coenzyme Q10.

4. Procédé de fabrication selon la revendication 1, **caractérisé en ce que** l'antioxydant est choisi à partir d'un ou plusieurs éléments parmi le gallate de propyle, le BHT, le polyphénol de thé, 2l'α-tocophérol, le 6-palmitate d'acide L-ascorbique, le palmitate de polyphénol de thé, l'ascorbate de sodium, l'acide ascorbique, le thiodipropionate de dilauryle et l'acide lipoïque ; et de préférence, l'antioxydant est un antioxydant hydrosoluble comprenant un ou plusieurs éléments parmi l'acide ascorbique, l'ascorbate de sodium, l'acide érythorbique et l'érythorbate de sodium.

5. Procédé de fabrication selon la revendication 1, **caractérisé en ce que** le matériau de paroi est constitué d'un colloïde hydrosoluble et d'un glucide.

6. Procédé de fabrication selon la revendication 5, **caractérisé en ce que** le colloïde hydrosoluble est choisi à partir d'un ou plusieurs éléments parmi la gélatine, la gomme arabique, l'amidon modifié gélatinisable et l'octényle succinate d'amidon ; et le glucide est choisi à partir d'un ou plusieurs éléments parmi la dextrine, le glucose, le sucre granulé blanc, le fructose, le maltose, l'inositol et l'amidon de maïs.

7. Procédé de fabrication selon la revendication 1, **caractérisé en ce que** l'émulsionneur de cavitation en série à plusieurs étages est un émulsionneur de cavitation en série comportant plus de trois étages ; et de préférence, l'émulsionneur de cavitation en série à plusieurs étages est un émulsionneur de cavitation en série comprenant de 5 à 10 étages.

8. Procédé de fabrication selon la revendication 1, **caractérisé en ce que** chaque étage de l'émulsionneur de cavitation est constitué d'une section de contraction et d'une section d'expansion qui sont en communication, et une sortie de la section de constriction et une sortie de la section d'expansion ne se chevauchent pas entièrement ou partiellement dans une direction de sortie de la section de constriction.
